# EUROPEAN PATENT SPECIFICATION

(11) **EP 1 596 894 B1**
(45) Date of publication and mention of the grant of the patent: **28.06.2006**
(21) Application number: 04714087.6
(22) Date of filing: 24.02.2004
(51) Int. Cl.: A61L 31/02, A61M 25/01

(54) **COMPOSITE MEDICAL DEVICE**
MEDIZINISCHE VERBUNDVORRICHTUNG
DISPOSITIF MEDICAL COMPOSITE

(30) Priority: 26.02.2003 US 375766
(43) Date of publication of application: 23.11.2005
(73) Proprietor: Boston Scientific Limited, St. Michael (BB)
(72) Inventor: REYNOLDS, Brian, R., Ramsey, MN 55303 (US); SKUJINS, Peter, Minneapolis, MN 55403 (US)
(74) Representative: Vossius & Partner
(86) International application number: PCT/US2004/005343
(87) International publication number: WO 2004/075941

(56) References cited:
- EP-A- 0 689 849
- EP-A- 0 806 220
- EP-A- 0 838 230
- EP-A- 1 287 846
- WO-A-00/40286
- WO-A-03/030982
- US-A- 5 238 004
- US-A- 5 772 609

## Description

### Field of the Invention

The invention generally pertains to medical devices, and more specifically to composite medical devices including two or more structural elements connected together, and a method of making the same.

### Background

A wide variety of medical devices, such as guidewires, catheters, and the like, have been developed for use in facilitating navigation and treatment throughout the anatomy of a patient. Because the anatomy of a patient may be very tortuous, it is desirable to combine a number of performance features in such a medical device. It is generally known to provide medical devices including multiple structural elements connected together to provide a number of performance features in a medical device. The prior art offers a number of different structures and mechanisms for connecting structural elements in medical devices. Each of these different structures and mechanisms has certain advantages and disadvantages. However, there is an ongoing need to provide alternative medical device structures and assemblies.

### Summary

The invention provides several alternative designs, materials and methods of manufacturing alternative medical device structures and assemblies.

### Brief Description of the Drawings

The invention may be more completely understood in consideration of the following detailed description of various embodiments of the invention in connection with the accompanying drawings, in which:
Figure 1 is cross sectional fragmentary view of a medical device shown as a guidewire (pre-grinding), including a connection utilizing an overlapping tapered joint and a tubular connector for joining a proximal section and a distal section of the guidewire;
Figure 2 is a cross sectional fragmentary view of the guidewire (post grinding) of Figure 1;
Figure 3 is a cross sectional fragmentary view of an alternative guidewire (post grinding), including a connection utilizing a butt joint and a tubular connector for joining a proximal section and a distal section of the guide wire;
Figure 4 is a cross sectional fragmentary view of an alternative guidewire (post grinding), including a connection utilizing an overlapping joint and a tubular connector for joining a proximal section and a distal section of the guide wire;
Figures 5A-5C are cross sectional fragmentary views of various end portions for use with the guidewire embodiment of Figure 4; and
Figure 6 is a cross sectional fragmentary view of an alternative guidewire, including a connection utilizing a joint and a tubular connector for joining a proximal section and a distal section of the guide wire, wherein there is spacing between the the proximal section and the distal section of the guide wire.

While the invention is amenable to various modifications and alternative forms, specifics thereof have been shown by way of example in the drawings and will be described in detail.

### Detailed Description of the Invention

For the following defined terms, these definitions shall be applied, unless a different definition is given in the claims or elsewhere in this specification.

Weight percent, percent by weight, wt%, wt-%, % by weight, and the like are synonyms that refer to the concentration of a substance as the weight of that substance divided by the weight of the composition and multiplied by 100.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The detailed description and drawings illustrate examples of various embodiments of the claimed invention, and are not intended to be limiting.

At least some embodiments of the invention provide a medical device, or components or structures for use in a medical device; that include two or more structural elements that are connected using a bismuth alloy connector material. The bismuth alloy connector material is configured to expand when solidified. Some embodiments include a structural member defining an opening therein, and another component or structure that includes a portion that extends into the opening. The bismuth alloy connector material is also present within the opening, and upon solidification, the bismuth alloy connector material expands to exert a compressive force within the opening. The compressive force acts to connect the structural member to the other component or structure by exerting the compressive force on the inner surface of the opening of the structural member and on the portion of the other component or structure that extends into the opening. A mechanical interlock is thereby provided between the structural member and the other component or structure.

In some embodiments, the use of such a bismuth alloy connector material can provide for some advantages. For example, in some embodiments, because the connection provided by the using such material does not depend on alloying with, or chemically adhering to the materials of the components being connected, there is less of a concern regarding providing surfaces to be attached that are absolutely free of contaminants or surface oxides. Thus, where conventional preparation of surfaces of some materials that are to be connected may require a substantial amount of treatment or preparation, many such treatments or preparations are not necessary in at least some embodiments using a bismuth alloy connector material. This can be advantageous for many reasons, especially in situations where the components to be connected are difficult to expose to such treatments. For example, some components may be very small, or may include portions that are physically difficult to reach with such treatments or preparations. Additionally, some components may be made of materials that do not react well with , or cannot be exposed to harsh preparation or treatment methods.

In some embodiments, the structural member is a connector member that is particularly adapted and configured to interconnect two or more other components or structures. For example, the structural member may be a connector tube that is particularly adapted and configured to have portions of other structures, such as the ends of elongated members such as wires or hypotubes, inserted therein for connection using the bismuth alloy connector material. In some other embodiments, the structural member is a component of the medical device that includes an opening or aperture on a portion thereof that is adapted and configured for use in connecting the structural member with other components of the device using the bismuth alloy connector material. For example, the structural member may be a wire or hypotube defining an opening or lumen in the end thereof, or other such structure.

Although discussed with specific reference to guidewires in much of the description below, the invention may be applicable to almost any medical device. For example, the invention may be applicable to shafts for catheters (e.g., guide catheters, balloon catheters, stent delivery catheters, etc.), infusion devices, distal protection devices, or shafts for rotational devices (atherectomy catheters, IVUS catheters, etc.). In some embodiments, the medical device is particularly adapted and configured for use in intravascular applications.

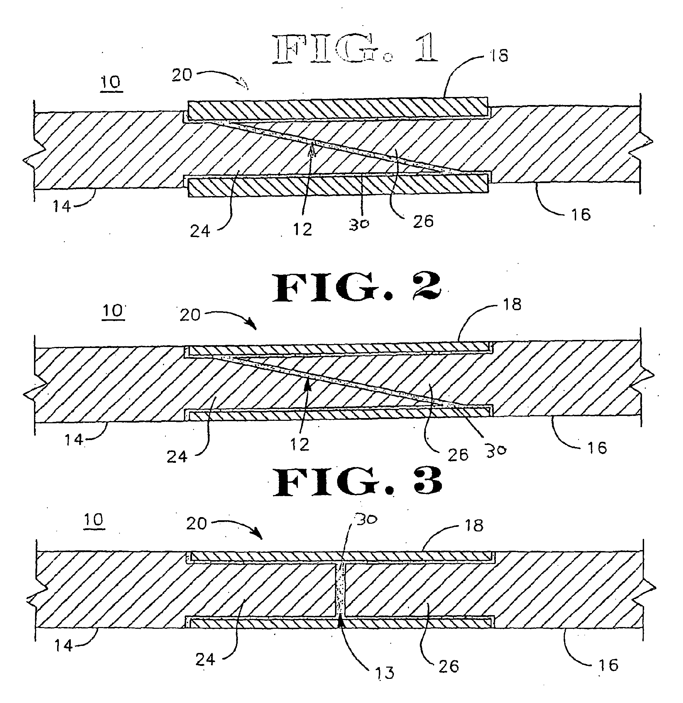

Refer now to Figures 1-4 which illustrate cross sectional views of a portion of a guidewire 10 including a connection 20 joining a proximal guidewire section 14 and a distal guidewire section 16. Figure 1 illustrates the guidewire 10 and the connection 20 before a final grinding step, and Figure 2 illustrates the guidewire 10 and the connection 20 after the final grinding step, which provides a smooth outer profile. The embodiment of Figures 1 and 2 utilizes an overlapping tapered joint 12, a connector structure 18 and a bismuth alloy connector material 30.

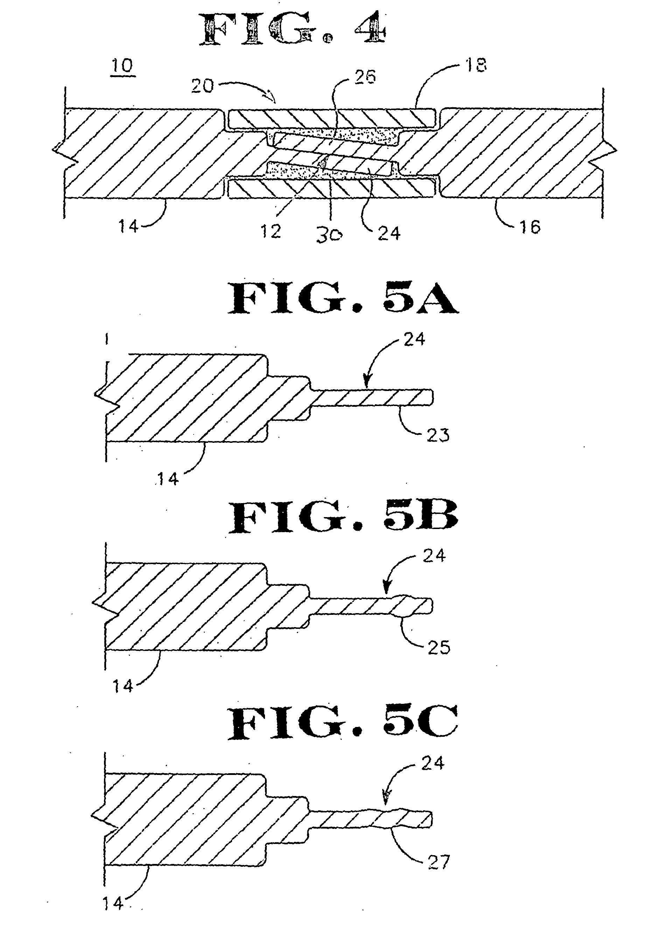

The embodiment of Figure 3 is similar to the embodiment of Figures 1 and 2, except that the connection 20 between the proximal guidewire section 14 and the distal guidewire section 16 does not utilize an overlapping joint 12, but rather uses a butt joint 13. The embodiment of Figure 4 is also similar to the embodiment of Figures 1 and 2, except that the connection 20 between the proximal guidewire section 14 and the distal guidewire section 16 utilizes an overlapping joint 12 that is not tapered.

Those of skill in the art and others will recognize that the materials, structure, and dimensions of the proximal/distal guidewire sections 14/16 are dictated primary by the desired characteristics and function of the final guidewire, and that any of a broad range of materials, structures, and dimensions can be used.

For example, the proximal and distal guidewire sections 14/16 may have a solid cross-section as shown, or a hollow cross-section, and may be formed of any materials suitable for use, dependent upon the desired properties of the guidewire. Some examples of suitable materials include metals, metal alloys, and polymers. In some embodiments, it is desirable to use metals, or metal alloys that are suitable for metal joining techniques such as welding, soldering, brazing, crimping, friction fitting, adhesive bonding, etc. As used herein, the proximal section 14 and the distal section 16 may generically refer to any two adjacent guidewire sections along any portion of the guidewire.

In some embodiments, the proximal guidewire section 14 may be formed of relatively stiff material such as straightened 304v stainless steel wire. Alternatively, proximal portion 14 may be comprised of a metal or metal alloy such as a nickel-titanium alloy, nickel-chromium alloy, nickel-chromium-iron alloy, cobalt alloy, a polymer material, such as a high performance polymer, or other suitable material, or the like. In general, the material used to construct proximal portion 14 may be selected to be relatively stiff for pushability and torqueability.

In some embodiments, the distal guidewire section 16 may be formed of a relatively flexible material such as a straightened super elastic (i.e. pseudoelastic) or linear elastic alloy (e.g., nickel-titanium) wire, or a alternatively, a polymer material, such as a high performance polymer, or similar such material or the like. Alternatively, distal portion 16 may be comprised of a metal or metal alloy such as stainless steel, nickel-chromium alloy, nickel-chromium-iron alloy, cobalt alloy, or other suitable material. In general, the material used to construct distal portion 16 may be selected to be relatively flexible for trackability.

In some particular embodiments, the distal section 16 is a linear elastic nickel-titanium alloy, for example, linear elastic nitinol. The word nitinol was coined by a group of researchers at the United States Naval Ordinance Laboratory (NOL) who were the first to observe the shape memory behavior of this material. The word nitinol is an acronym including the chemical symbol for nickel (Ni), the chemical symbol for titanium (Ti), and an acronym identifying the Naval Ordinance Laboratory (NOL).

Within the family of commercially available nitinol alloys, is a category designated "linear elastic" which, although is similar in chemistry to conventional shape memory and superelastic varieties, exhibits distinct and useful mechanical properties. By skilled applications of cold work, directional stress, and heat treatment, the wire is fabricated in such a way that it does not display a substantial "superelastic plateau" or "flat region" in its stress/strain curve. Instead, as recoverable strain increases, the stress continues to increase in an essentially linear relationship until plastic deformation begins. In some embodiments, the linear elastic nickel-titanium alloy is an alloy that does not show any martensite/austenite phase changes that are detectable by DSC and DMTA analysis over a large temperature range. For example, in some embodiments, there is no martensite/austenite phase changes detectable by DSC and DMTA analysis in the range of about -60°C to about 120°C. The mechanical bending properties of such material are therefore generally inert to the effect of temperature over this very broad range of temperature. In some particular embodiments, the mechanical properties of the alloy at ambient or room temperature are substantially the same as the mechanical properties at body temperature. In some embodiments, the use of the linear elastic nickel-titanium alloy for the distal portion 16 allows the guidewire to exhibit superior "pushability" around tortuous anatomy.

In some embodiments, the linear elastic nickel-titanium alloy comprises in the range of about 50 to about 60 wt.% nickel, with the remainder being essentially titanium. In some particular embodiments, the composition comprises in the range of about 54 to about 57 wt. % nickel. One example of a suitable nickel-titanium alloy is FHP-NT alloy commercially available from Furukawa Techno Material Co. of Kanagawa, Japan. Some examples of nickel-titanium alloys are disclosed in U.S. Patent Nos. 5,238,004 and 6,508,803.

In some particular embodiments, the proximal guidewire section 14 is formed from a stainless steel wire having a diameter in the range of 0.01 to 0.02 inches, and a length in the range of about 50 to about 110 inches, and the distal guidewire section 16 is formed from a linear elastic nitinol wire having a diameter that ranges from a diameter to match the diameter of the proximal guidewire section 14 to as small as about 0.002 inches, and a length in the range of 3 to 15 inches.

The distal end 24 of the proximal portion 14 and the proximal end 26 of distal portion 16 (i.e., the joined ends) may form an overlapping tapered joint 12 as shown in Figures 1 - 2. Alternatively, the joined ends 24/26 may form a butt joint 13 as shown in Figure 3. The joined ends 24/26 in the butt joint 13 can be in direct contact with each other, or can include a degree of spacing between the joined ends, as shown in Figure 3. As a further alternative, the joined ends 24/26 may form an overlapping joint 12 that is not tapered as shown in Figure 4. The one or both of the non-tapered end portions 24/26 may have a uniform profile (diameter) 23 as shown in Figure 5A, a bulbous portion 25 for purposes of mechanical interlocking as shown in Figure 5B, or a helical form 27 for purposes of mechanical interlocking as shown in Figure 5C. Additionally, in some embodiments, using either an overlapping type joint or using a butt type joint, portions of the outer surfaces of either of the joined ends can be provided with additional structures, such as grooves, ridges, a roughened or textured surface, or the like for the purpose of providing better mechanical interlocking between the joined ends and the connector structure or connector material.

In each of the embodiments illustrated in Figures 1 - 2 and 4, the end portions 24/26 overlap to form an overlapping joint 12. The overlapping joint 12 can act to blend the stiffness of proximal portion 14 and distal portion 16, if desired, by combining the properties of each end section 24/26 making up the cross section of the overlapping joint 12. Thus, the joint 12 forms a flexibility transition region that has a relative flexibility that is between the flexibility of the proximal portion 14 and the flexibility of the distal portion 16.

In the tapered embodiments illustrated in Figures 1 - 2, the ends 24/26 may be tapered or otherwise formed to have a mating geometry that gradually decreases in cross sectional area toward the middle of the connection 20. The tapered overlapping portion 12 may define a uniform or a non-uniform transition of the sections 24/26, depending on the transition characteristics desired. For example, the end sections 24/26 may be linearly tapered as shown, tapered in a curvilinear fashion, or tapered in a step-wise fashion. If tapered linearly as shown, the angle of the taper may vary. Using the longitudinal center axis of the guidewire 10 as a reference, as measured from the extreme ends of the end sections 24/26, the angle of the taper is acute (i.e., less than 90 degrees), and may be in the range of 5 degrees to 45 degrees, for example. Varying the angle of the tapered ends 24/26 also varies the length of the overlapping joint 12 in accordance with geometric principles. The length of the overlapping joint 12 may be selected to obtain a more (longer length) or less (shorter length) gradual transition in stiffness.

As mentioned previously, the proximal guidewire section 14 and the distal guidewire section 16 may be formed of different materials (i.e., materials having different moduli of elasticity) resulting in a difference in flexibility. For example, the proximal guidewire section 14 may be formed of stainless steel wire and the distal guidewire section 16 may be formed of nickel-titanium alloy wire, both having the same dimensions, resulting in a 3:1 difference in elastic modulus. Such a difference in elastic modulus (i.e., flexibility) may result in a stress concentration point during flexure and/or torsion that may have a tendency to kink and fracture. By virtue of the gradual transition in stiffness provided by the overlapping portion 12, stress is distributed along the entire length of the connection 20 thereby decreasing the probability that guidewire 10 may kink at the junction.

A gradual transition in stiffness may also allow the connection 20 to be located further distally. According to this embodiment, the distal portion 16 may be manufactured to be shorter than proximal portion 14. Including a relatively long proximal section 14 may advantageously increase the torquability and pushability of the guidewire 10. Although only one connection 20 is shown, additional connections 20 may be used to connect other guidewire sections of varying stiffness.

The connector structure 18 may comprise a structure defining one or more openings therein or one or more lumens extending there through. In some embodiments, the connector structure 18 is a generally tubular structure such as a hypotube as shown, or a coiled wire, or the like. The connector 18 may have an inside diameter sized and shaped appropriately to receive the ends 24/26 of the proximal portion 14 and the distal portion 16, and an outside diameter sufficient to accommodate a final grinding procedure. In the embodiment shown, the outside surface of the connector structure is generally circular in cross-sectional shape, however, other geometries, for example, oval, or multisided geometries may be used in other embodiments. In some embodiments, the connector 18 can include one or more grooves, slits, slots, or the like, that are defined in the body of the connector, for example, to provide a desired degree of flexibility characteristics to the connector 18. Some examples of such structures are disclosed in a U.S. Patent Application entitled "ARTICULATING INTRACORPORAL MEDICAL DEVICE" (Attorney docket no. 1001.1668101) filed on even date herewith. Some other examples of suitable techniques and structures that can be used to interconnect different shaft sections are disclosed in U.S. Patent Application Nos. 09/972,276 filed on October 5, 2001 and 10/068,992 filed on February 28, 2002. Some additional examples of structures and materials that can be used in medical device constructions are also disclosed in a U.S. Patent Application entitled "ELONGATED INTRACORPORAL MEDICAL DEVICE" (Attorney docket no. 1001.1673101) filed on even date herewith.

In some example embodiments, the connector 18 is generally tubular, and can have an inner diameter in the range of 0.127 (0.005) to 0.508 mm (0.02 inches), and an outer diameter in the range of 0.254 (0.01) to 0.635 mm (0.025 inches). In some particular embodiments, the connector 18 can have an inner diameter of about 0.254 (0.010) mm (inches) and an outer diameter of about 0.014 inches. The final diameter of the guidewire 10 and the connector 18 may be in the range of 0.254 (0.010) to 0.457mm (0.018 inches) for example. By way of example, not limitation, the connector 18 may have a length of about 2.54 (1.0) to 7.62 cm (3.0 inches) for an overlapping portion 12 of about 6.35 (0.25) to 63.5 mm (2.5 inches). However, in some other embodiments, this type of construction can be applied to wires or other structures of larger diameter intended, for example, for peripheral intervention purposes. Such wires could range as large as .035 in diameter and therefore have an extended length connector and correspondingly longer overlapping sections.

The connector 18 may include or be made of a metal or metal alloy, and may include radiopaque materials. Suitable metals and metal alloys include stainless steels, nickel-titanium alloys (e.g., nitinol), nickel-chromium alloys, nickel-chromium-iron alloys, cobalt alloys, nickel, or other suitable materials, and the like. An example is a nickel-chromium-iron alloy designated UNS N06625 and is available under the trade name INCONEL 625, which may be obtained from California Fine Wire Company of Grover Beach, California. One example of a nickel-molybdenum-chromium alloy is designated UNS 10276 and is available under the trade name ALLOY C276 from Fort Wayne Metals Research Products Corporation of Fort Wayne, Indiana. An example of a nickel-molybdenum alloy are those of the Hastelloy family and an example of which is available under the trade name ALLOY B2 from Fort Wayne Metals Research Products Corporation of Fort Wayne, Indiana. Alternatively, connector 18 may be comprised of a polymer or a metal-polymer composite, and the like.

As indicated above, the connector 18, or portions thereof can also be made of or include a radiopaque material. Radiopaque materials are understood to be materials capable of producing a relatively bright image on a fluoroscopy screen or another imaging technique during a medical procedure. This relatively bright image aids the user of device 10 in determining its location. Some examples of radiopaque materials can include, but are not limited to, gold, platinum, palladium, tantalum, tungsten alloy, plastic material loaded with a radiopaque filler, and the like.

It should also be understood that in some embodiments, the connector structure, or the structure defining one or more openings therein, may be part of, or integral with, one of the structural elements being connected together. For example, the connector structure could be defined by an end of a wire or hypotube that includes an opening, recess, or lumen defined therein, into which the bismuth alloy connector material and the end of another structure are inserted for connection thereto. Additionally, the connector structure may be attached to one of the structures being connected together using more conventional techniques, such as adhesive bonding, thermal bonding, soldering, brazing, welding, mechanical connection (e.g. crimping, friction fitting, etc...), while being connected to another structures using the bismuth alloy connector material as discussed herein.

The bismuth alloy connector materials, for example connector material 30, for use in making the connection are adapted and configured to have the characteristic of expansion or growth upon or after solidification. In at least some embodiments, although normal thermal shrinkage of the liquid bismuth alloy may take place during cooling of the liquid, the crystalline structure that forms during solidification occupies a larger volume than the same mass of liquid. When the bismuth alloy connector materials are confined within a predetermined space, for example within an opening in a connector structure, compressive forces are generated as the alloy solidifies and expands or grows within the space.

Some examples of suitable bismuth alloy connector materials include alloys of bismuth including additional alloying elements such as tin, indium, cadmium, lead, and the like. Due to the fact that the final medical device will make contact with or be inserted into a living body, in some embodiments, the alloy should include only elements that are known to be acceptable for contact with the body. For example, bismuth alloys including elements such as tin, indium, or the like, may be more acceptable for contact with the body. Some example alloys can include in the range of about 4 to about 80 wt. % bismuth, with the remainder being other alloying elements. Some examples of suitable alloys, and example ranges of wt. % of components within some alloys, and some specific examples of such alloys, include those having the ranges of components as illustrated in Table 1 as follows:

**Table 1**

| **Type of alloy:** | **Range of components in some embodiments:** | **One example of a specific alloy falling within these ranges:** |
|---|---|---|
| **Bismuth-Tin alloy** | 35 to 45 wt. % Bi, and | 40 wt. Bi, and |
| | 55 to 65 wt. % Sn | 60 wt. % Sn |
| **Bismuth-Tin alloy** | 53 to 63 wt. % Bi, and | 58 wt. % Bi, and |
| | 37 to 47 wt. % Sn | 42 wt. % Sn |
| **Bismuth-Indium alloy** | 2 to 10 wt. % Bi, and | 5 wt. % Bi, and |
| | 90 to 98 wt. % In | 95 wt. % In |
| **Bismuth-Indium alloy** | 62 to 72 wt. % Bi, and | 67 wt. % Bi, and |
| | 28 to 38 wt. % In | 33 wt. % In |
| **Bismuth-Indium alloy** | 29 to 39% Bi, and | 34 wt. % Bi, and |
| | 61 to 71 wt. % In | 66 wt. % In |
| **Bismuth-Indium-Tin alloy** | 53 to 63 wt. % Bi, | |
| | 20 to 30 wt. % In, and | |
| | 12 to 22 wt. % Sn | |

At least some of the bismuth alloys that can be used as the connector material are characterized by relatively low melting temperatures compared to some other metal alloys. For example, in some embodiments, the bismuth alloy used is characterized as being a "fusible" alloy, meaning that it has a melting point in the range of about 50 to about 260°C. In some embodiments, the alloy has a melting point in the range of about 200°C or below, or in the range of about 150°C or below. In some embodiments, the alloy used is the eutectic alloy for the particular type of alloy being used, meaning that it is the particular alloy having the lowest melting point (i.e. eutectic point) that is obtainable by varying the proportions of the components of the alloy. Eutectic alloys have definite and minimum melting points in contrast to other combinations of the same metals. For such eutectic alloys, the minimum melting points as given above would be the eutectic melting point.

The low melting temperatures can be useful when the connector material is used in conjunction with structural elements that are made of or include temperature sensitive material. For example, some nickel-titanium alloys are annealed or shape set by exposure to higher temperatures. Therefore, the use of alloys having a lower temperature melting point can help to preserve the desired heat-treat state of structures made of such nickel-titanium alloys that need to be connected to other structures.

To manufacture the connection 20 of the guidewire 10, the ends 24/26 of the proximal and distal guidewire sections 14/16 may be ground or otherwise worked to form the desired size and shape (e.g., uniform diameter 23, bulbous portion 25, helix 27, taper, or mechanical interlocking features, such as grooves, ridges, roughened surfaces, etc.) to accommodate the size and shape of the connector structure 18, or to accommodate the type of joint being constructed (e.g. an overlapping joint, a butt joint, etc.). Additionally, the size and shape of the ends 24/26 and the connector structure 18 can be configured to allow for the inclusion of the bismuth alloy connector material 30, and for the flow of the connector material in a liquid state. A recess step may be ground into the proximal and distal guidewire sections 14/16 to accommodate the connector structure, such as the connector tube 18.

The ends 24/26 of the proximal and distal guidewire sections 14/16 and the bismuth alloy connector material 30 are disposed within the lumen defined in the connector structure 18 in such a manner that the bismuth alloy connector material 30 solidifies and expands to exert a compressive force within the lumen. The compressive force within the lumen acts to connect the connector structure 18 to the ends 24/26 of the proximal and distal guidewire sections 14/16, and thereby connect the proximal and distal guidewire sections 14/16 to each other. The ends 24/26 of the proximal and distal guidewire sections 14/16 and the bismuth alloy connector material 30 can be disposed within the lumen of the connector structure 18 using any suitable process or method that allows for such a connection to be formed.

For example, in some embodiments, a sufficient amount of the bismuth alloy connector material is applied to either one or both ends 24/26 of the proximal and distal guidewire sections 14/16, and bismuth alloy connector material is allowed to solidify. The bismuth alloy connector material can be applied to the ends 24/26 using any suitable process, for example, a hot dipping process, a coating process, a spraying process, a plating process, or the like. The ends 24/26 are then inserted into the lumen within the connector tube 18 until a dimensional interference is created. The bismuth alloy connector material is then heated above its melting point of the bismuth alloy connector material, and an additional insertion force is applied to the guidewire sections 14/16 to provide axial movement of the ends 24/26 further into the connector structure and into a bonding position. For example, the distal end 24 of the proximal portion 14 and proximal end 26 of the distal portion 16 can be positioned adjacent one another in an overlapping 12 or an end-to-end 13 arrangement within the connector structure 18. In some embodiments, when ends 24/26 are moved into the bonding position, some excess bismuth alloy connector material may be displaced from within the lumen in the connector structure 18, indicating that the opening or lumen if full to capacity. The bismuth alloy connector material 30 is allowed to cool and solidify within the lumen. As the bismuth alloy connector material solidifies, it expands to exert a compressive force within the lumen. The compressive force within the lumen acts to provide a mechanical interlock between the connector structure 18 to the ends 24/26 of the proximal and distal guidewire sections 14/16, and thereby connect the proximal and distal guidewire sections 14/16 to each other.

Additionally, in some embodiments, as a result of the bismuth alloy expansion within the lumen, there-may be a slight amount of outward motion of the guidewire sections 14/16 from the lumen of the connector structure 18. In some embodiments, since the amount of expansion is very predictable and consistent, the wire components can be sized appropriately to compensate for this. Additionally, the bismuth alloy that may solidify outside of the connector 18 can serve to form or function as strain relief just proximal and distal of the connector. In some embodiments, bismuth alloy that may solidify outside of the connector 18 may have a constant diameter, which can be beneficial for strain relief.

Alternatively, a sufficient amount of the bismuth alloy connector material can be applied to either one or both ends 24/26 of the proximal and distal guidewire sections 14/16, and either one or both ends 24/26 are inserted into a bonding position within the lumen within the connector tube 18 prior to solidification of the bismuth alloy connector material. The bismuth alloy connector material 30 can then be allowed to cool, solidify, and expand to provide a connection as discussed above.

Another alternative method can entail disposing the bismuth alloy connector material within the lumen of the connector structure 18 prior to insertion of the ends 24/26 into the lumen. In some such embodiments, the ends 24/26 can be inserted in a bonding position within the lumen prior to solidification of the bismuth alloy connector material. In some other such embodiments, the bismuth alloy connector material can be allowed to solidify, and is then reheated prior to or during the insertion of the ends 24/26 into a bonding position within the lumen. Again, the bismuth alloy connector material 30 can then be allowed to cool, solidify, and expand to provide a connection as discussed above.

In most cases, a permanent connection (as opposed to a releasable connection) is made. However, due to the nature of the bismuth alloy connector material, the joint can be disconnected, or reworked by reheating the connector material and separating or reworking the components of the joint.

Once connected, the connector tube 18 and the proximal and distal guidewire sections 14/16 can be worked or formed to provide desired characteristics, such as shape or flexibility characteristics. For example, connector tube 18 and the proximal and distal guidewire sections 14/16 can be centerless ground to provide a smooth and uniform profile across the connection 20, and to straighten out small misalignments between the proximal and distal guidewire sections 14/16. Other portions of the guidewire 10 may be ground as well to provide the desired tapers and changes in diameter. For example, one or both of the proximal and distal guidewire sections 14/16 can be continuously tapered, can have a tapered section or a number or series of tapered sections of differing diameters, or can have a constant diameter. In some embodiments, the sections 14/16 are tapered or otherwise formed to have a geometry that decreases in cross sectional area toward the distal end thereof. If tapered, the sections 14/16 can include a uniform or a non-uniform transition of the sections, depending on the transition characteristics desired. For example, one or both of the sections 14/16 may be linearly tapered, tapered in a curvilinear fashion, or tapered in a step-wise fashion. The angle of any such tapers can vary, depending upon the desired flexibility characteristics. The length of the taper may be selected to obtain a more (longer length) or less (shorter length) gradual transition in stiffness. The centerless grinding technique may utilize an indexing system employing sensors (e.g., optical/reflective, magnetic) to avoid excessive grinding of the connection 20. In some embodiments, the presence of dissimilar materials in the construction can influence the grinding technique and tooling used to accomplish uniform material removal, create smooth transitions, and successfully bridge across adjacent components. In addition, the centerless grinding technique may utilize a CBN or diamond abrasive grinding wheel that is well shaped and dressed to avoid grabbing the connector 20 during the grinding process.

Once finally ground or otherwise worked or shaped, in some embodiments, a flexible coil (such as a coil tip) and/or a polymer jacket (such as a polymer tip) (optionally covering connection 20) or combination thereof, and other such structure, such as radiopaque markers, safety and/or shaping ribbons (coiled or uncoiled), and the like, may be placed on the guidewire 10, for example, adjacent the distal portion. Some examples of guidewire constructions, for example tip constructions, are disclosed in U.S. Patent Application Number 10/068,992 filed February 28, 2002, entitled "Composite Guidewire". Additionally, in some embodiments, a coating, for example a lubricious (e.g., hydrophylic) or other type of coating may be applied to all or portions of the guidewire. Different coatings can be applied to different sections of the guidewire. Some examples of such coatings and materials and methods used to create such coatings can be found in U.S. Patent Nos. 6,139,510 and 5,772,609.

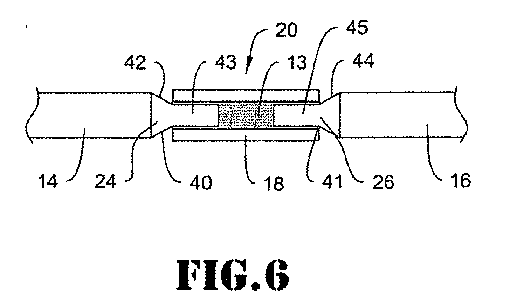

Refer now to Figure 6, which illustrates another example embodiment similar to the embodiment of Figure 3, except that the connection 20 between the proximal guidewire section 14 and the distal guidewire section 16 uses a butt type joint 13, but wherein the joined ends 24/26 are spaced from one another. Additionally, in the embodiment of Figure 6, the joined ends 24/26 include reduced diameter portions 40 and 41. The reduced diameter portion 40 includes tapering portion 42 and constant diameter portion 43. Reduced diameter portion 41 includes tapering portion 44 and constant diameter portion 45. The constant diameter portions 43 and 45 are configured to fit within the connector 18, and are attached to the connector 18 using a bismuth fusible alloy material, as discussed above. Some additional examples of structures and materials that can be used in medical device constructions are also disclosed in a U.S. Patent Application entitled "ELONGATED INTRACORPORAL MEDICAL DEVICE" (Attorney docket no. 1001.1673101) filed on even date herewith.

It should be understood that this disclosure is, in many respects, only illustrative. Changes may be made in details, particularly in matters of shape, size, and arrangement of steps without exceeding the scope of the invention. For example, alternative structure can be used in connecting the proximal and distal sections of medical devices. Additionally, alternative tip constructions including a flexible coil tip, a polymer jacket tip, a tip including a coiled safety/shaping wire, or combination thereof, and other such structure may be placed on the medical device. The invention's scope is, of course, defined in the language in the claims.

## Claims

1. A medical device, comprising:
an elongated section having an end;
a structural member defining an opening therein, wherein the end of the elongated section extends into the opening; and
a bismuth alloy connector material disposed within the opening, the connector material configured to expand when solidified to exert a compressive force within the opening in the structural member.

2. The medical device of claim 1, wherein the bismuth alloy connector material is solidified and has expanded to exert a compressive force on the structural member and the end of the elongated section to provide a mechanical interlock between the structural member and the elongated section.

3. The medical device of claim 1, wherein the structural member defines a second opening therein, and the medical device further includes a second elongated section having an end, wherein the end of the second elongated section extends into the second opening; and
a bismuth alloy connector material disposed within the second opening, the connector material configured to expand when solidified to exert a compressive force within the structural member.

4. The medical device of claim 3, wherein the first opening and the second opening are openings at the opposite ends of a common lumen defined in the structural member.

5. A medical device of claim 4, wherein the first elongated section comprises a proximal section having a distal end, the second elongated section comprises a distal section having a proximal end; and the structural member comprises a connector member defining the lumen having the first opening and the second opening, wherein the distal end of the proximal section extends into the lumen through the first opening and the proximal end of the distal section extends into the lumen through the second opening, and the bismuth alloy connector material is disposed within the lumen, and the connector material is configured to expand when solidified to exert compressive forces within the connector member.

6. The medical device of any of claims 1-5, wherein the bismuth alloy connector material has a eutectic melting point in the range of 200°C or below, or alternatively, the bismuth alloy connector material has a eutectic melting point in the range of 150°C or below.

7. The medical device of any of claims 1-6, wherein the bismuth alloy connector material comprises in the range of 4 to 80 wt. % bismuth.

8. The medical device of any of claims 1-7, wherein the bismuth alloy connector material comprises bismuth and an additional alloying element selected from tin, indium, or mixtures thereof.

9. The medical device of any of claims 1-8, wherein the bismuth alloy connector material comprises in the range of 35 to 45 wt. % bismuth, and in the range of 55 to 65 wt. % tin, or alternatively, the bismuth alloy connector material comprises in the range of 53 to 63 wt. % bismuth, and in the range of 37 to 47 wt. % tin.

10. The medical device of claim any of claims 1-9, wherein the bismuth alloy connector material comprises a eutectic bismuth-tin alloy.

11. The medical device of any of claims 1-8, wherein the bismuth alloy connector material comprises in the range of 2 to 10 wt. % bismuth, and in the range of 90 to 98 wt. % indium, or alternatively, the bismuth alloy connector material comprises in the range of 62 to 72 wt. % bismuth, and in the range of 28 to 38 wt. % indium, or alternatively, the bismuth alloy connector material comprises in the range of 29 to 39 wt. % bismuth, and in the range of 61 to 71 wt. % indium.

12. The medical device of any of claims 1-8, or 11, wherein the bismuth alloy connector material comprises a eutectic bismuth-indium alloy.

13. The medical device of any of claims 1-8, wherein the bismuth alloy connector material comprises in the range of 53 to 63 wt. % bismuth, in the range of 20 to 30 wt. % indium, and in the range of 12 to 22 wt. % tin.

14. The medical device of any of claims 1-8, or 13, wherein the bismuth alloy connector material comprises a eutectic bismuth-indium-tin alloy.

15. The medical device of claim 5, wherein the bismuth alloy connector material is solidified and has expanded to exert a compressive force on the connector member and the distal end of the proximal section and the proximal end of the distal section to provide a mechanical interlock between the connector member and the ends of the elongated sections.

16. A medical device of claim 5 or 15, wherein the proximal section has a first flexibility and the distal section has a second flexibility, and wherein the distal end of the proximal section and the proximal end of the distal section overlap to define a region that blends the first flexibility with the second flexibility.

17. A medical device of claim 5, 15, or 16, wherein the distal end of the proximal section has a reduced size, and the proximal end of the distal section has a reduced size.

18. A medical device of claim 17, wherein the reduced size portions have a uniform profile.

19. A medical device as in claim 17, wherein the reduced size portions have a taper.

20. A medical device as in claim 17, wherein the reduced size portions have an interlocking shape.

21. A medical device of claim 5 or 15, wherein the distal end of the proximal section and the proximal end of the distal section are joined to define a butt joint within the connector member.

22. A medical device of claim 5 or 15-20, wherein the distal end of the proximal section defines a tapered portion and the proximal end of the distal section defines a tapered portion, and the tapered portions at least partially overlap each other.

23. A medical device of any of claims 1-22, wherein the elongated section comprises a metal or metal alloy, and optionally, the metal or metal alloy comprises stainless steel, nickel-titanium alloy, nickel-chromium alloy, nickel-chromium-iron alloy, cobalt alloy, or combinations thereof.

24. A medical device of any of claims 5, or 15-22, wherein the medical device further includes an outer structure disposed about at least a portion of the distal section.

25. A medical device of any of claims 1-24, wherein the structural member comprises a metal or a metal alloy, and optionally, the metal or metal alloy comprises stainless steel, nickel-titanium alloy, nickel-chromium alloy, nickel-chromium-iron alloy, cobalt alloy, nickel, or combinations thereof.

26. A medical device of any of claims 1-24, wherein the structural member comprises a polymer or a metal-polymer composite.

27. A medical device of any of claims 5, or 15-22, wherein the connector structure comprises a tubular member disposed about the distal end of the proximal section and the proximal end of the distal section.

28. A medical device of any of claims 3-5, or 15-22, wherein the second elongated section comprises a metal or metal alloy, and optionally, the metal or metal alloy comprises stainless steel, nickel-titanium alloy, nickel-chromium alloy, nickel-chromium-iron alloy, cobalt alloy, or combinations thereof.

29. The medical device of any of claims 1-28, wherein the medical device comprises a guidewire.

30. A guidewire, comprising:
a proximal section having a distal end;
a distal section having a proximal end; and
a connector structure disposed adjacent the distal end of the proximal section and the proximal end of the distal section; and
means for exerting a compressive force on the connector structure and the ends of the proximal and distal sections to provide a mechanical interlock between the connector structure and the proximal and distal sections, the means including a bismuth alloy connector material.

31. A method of manufacturing the medical device of any of claims 1-29, the method comprising:
providing the elongated section having the end;
providing the structural member defining the opening therein;
disposing the end of the elongated section into the opening;
disposing the bismuth alloy connector material within the opening; and
allowing the bismuth alloy connector material to solidify and expand to exert a compressive force within the opening in the structural member.

32. A method of manufacturing the medical device of any of claims 3-5, 15-24, or 27-28, the method comprising:
providing the first elongated section having the end;
providing the second elongated section having the end;
providing the structural member defining the first opening and second opening therein;
disposing the end of the first elongated section into the first opening;
disposing the end of the second elongated section into the second opening;
disposing the bismuth alloy connector material within the first opening and the second opening; and
allowing the bismuth alloy connector material to solidify and expand to exert a compressive force within the first opening and the second opening in the structural member.

33. The method of claim 32, wherein the first opening and the second opening are openings at the opposite ends of a common lumen defined in the structural member.

34. The method of claim 32 or 33, wherein the medical device comprises a guidewire.

## Patentansprüche

1. Medizinische Vorrichtung mit:
einem länglichen Abschnitt mit einem Ende;
einem Strukturteil, das eine Öffnung darin bildet, wobei sich das Ende des länglichen Abschnitts in die Öffnung erstreckt; und
einem Verbindermaterial aus Wismutlegierung, das in der Öffnung angeordnet ist, wobei das Verbindermaterial so konfiguriert ist, daß es sich beim Erstarren ausdehnt, um eine Druckkraft in der Öffnung in dem Abschnitt auszuüben.

2. Medizinische Vorrichtung nach Anspruch 1, wobei das Verbindermaterial aus Wismutlegierung erstarrt ist und sich ausgedehnt hat, um eine Druckkraft auf das Strukturteil und das Ende des länglichen Abschnitts auszuüben, um für eine mechanische Verriegelung zwischen dem Strukturteil und dem länglichen Abschnitt zu sorgen.

3. Medizinische Vorrichtung nach Anspruch 1, wobei das Strukturteil eine zweite Öffnung darin bildet und die medizinische Vorrichtung ferner aufweist: einen zweiten länglichen Abschnitt mit einem Ende, wobei sich das Ende des zweiten länglichen Abschnitts in die zweite Öffnung erstreckt; und
ein Verbindermaterial aus Wismutlegierung, das in der zweiten Öffnung angeordnet ist, wobei das Verbindermaterial so konfiguriert ist, daß es sich beim Erstarren ausdehnt, um eine Druckkraft im Strukturteil auszuüben.

4. Medizinische Vorrichtung nach Anspruch 3, wobei die erste Öffnung und die zweite Öffnung Öffnungen an den entgegengesetzten Enden eines gemeinsamen Lumens sind, das im Strukturteil gebildet ist.

5. Medizinische Vorrichtung nach Anspruch 4, wobei der erste längliche Abschnitt einen proximalen Abschnitt mit einem distalen Ende aufweist, der zweite längliche Abschnitt einen distalen Abschnitt mit einem proximalen Ende aufweist; und das Strukturteil ein Verbinderteil aufweist, das das Lumen mit der ersten Öffnung und der zweiten Öffnung bildet, wobei sich das distale Ende des proximalen Abschnitts in das Lumen durch die erste Öffnung erstreckt, und sich das proximale Ende des distalen Abschnitts in das Lumen durch die zweite Öffnung erstreckt, und das Verbindermaterial aus Wismutlegierung im Lumen angeordnet ist und das Verbindermaterial so konfiguriert ist, daß es sich beim Erstarren ausdehnt, um Druckkräfte im Verbinderteil auszuüben.

6. Medizinische Vorrichtung nach einem der Ansprüche 1 bis 5, wobei das Verbindermaterial aus Wismutlegierung einen eutektischen Schmelzpunkt im Bereich von 200 °C oder darunter hat oder alternativ das Verbindermaterial aus Wismutlegierung einen eutektischen Schmelzpunkt im Bereich von 150 °C oder darunter hat.

7. Medizinische Vorrichtung nach einem der Ansprüche 1 bis 6, wobei das Verbindermaterial aus Wismutlegierung Wismut im Bereich von 4 bis 80 Gew.-% aufweist.

8. Medizinische Vorrichtung nach einem der Ansprüche 1 bis 7, wobei das Verbindermaterial aus Wismutlegierung Wismut und ein zusätzliches Legierungselement aufweist, das aus Zinn, Indium oder deren Mischungen ausgewählt ist.

9. Medizinische Vorrichtung nach einem der Ansprüche 1 bis 8, wobei das Verbindermaterial aus Wismutlegierung Wismut im Bereich von 35 bis 45 Gew.-% und Zinn im Bereich von 55 bis 65 Gew.-% aufweist oder alternativ das Verbindermaterial aus Wismutlegierung Wismut im Bereich von 53 bis 63 Gew.-% und zinn im Bereich von 37 bis 47 Gew.-% aufweist.

10. Medizinische Vorrichtung nach einem der Ansprüche 1 bis 9, wobei das Verbindermaterial aus Wismutlegierung eine eutektische Wismut-Zinn-Legierung aufweist.

11. Medizinische Vorrichtung nach einem der Ansprüche 1 bis 8, wobei das Verbindermaterial aus Wismutlegierung Wismut im Bereich von 2 bis 10 Gew.-% und Indium im Bereich von 90 bis 98 Gew.-% aufweist oder alternativ das Verbindermaterial aus Wismutlegierung Wismut im Bereich von 62 bis 72 Gew.-% und Indium im Bereich von 28 bis 38 Gew.-% aufweist oder alternativ das Verbindermaterial aus Wismutlegierung Wismut im Bereich von 29 bis 39 Gew.-% und Indium im Bereich von 61 bis 71 Gew.-% aufweist.

12. Medizinische Vorrichtung nach einem der Ansprüche 1 bis 8 oder 11, wobei das Verbindermaterial aus Wismutlegierung eine eutektische Wismut-Indium-Legierung aufweist.

13. Medizinische Vorrichtung nach einem der Ansprüche 1 bis 8, wobei das Verbindermaterial aus Wismutlegierung Wismut im Bereich von 53 bis 63 Gew.-%, Indium im Bereich von 20 bis 30 Gew.-% und Zinn im Bereich von 12 bis 22 Gew.-% aufweist.

14. Medizinische Vorrichtung nach einem der Ansprüche 1 bis 8 oder 13, wobei das Verbindermaterial aus Wismutlegierung eine eutektische Wismut-Indium-Zinn-Legierung aufweist.

15. Medizinische Vorrichtung nach Anspruch 5, wobei das Verbindermaterial aus Wismutlegierung erstarrt ist und sich ausgedehnt hat, um eine Druckkraft auf das Verbinderteil sowie das distale Ende des proximalen Abschnitts und das proximale Ende des distalen Abschnitts auszuüben, um für eine mechanische Verriegelung zwischen dem Verbinderteil und den Enden der länglichen Abschnitte zu sorgen.

16. Medizinische Vorrichtung nach Anspruch 5 oder 15, wobei der proximale Abschnitt eine erste Flexibilität hat und der distale Abschnitt eine zweite Flexibilität hat und wobei sich das distale Ende des proximalen Abschnitts und das proximale Ende des distalen Abschnitts überlappen, um einen Bereich zu bilden, in dem die erste Flexibilität und die zweite Flexibilität ineinander übergehen.

17. Medizinische Vorrichtung nach Anspruch 5, 15 oder 16, wobei das distale Ende des proximalen Abschnitts eine reduzierte Größe hat und das proximale Ende des distalen Abschnitts eine reduzierte Größe hat.

18. Medizinische Vorrichtung nach Anspruch 17, wobei die Teile mit reduzierter Größe ein gleichmäßiges Profil haben.

19. Medizinische Vorrichtung nach Anspruch 17, wobei die Teile mit reduzierter Größe eine Verjüngung haben.

20. Medizinische Vorrichtung nach Anspruch 17, wobei die Teile mit reduzierter Größe eine ineinandergreifende Form haben.

21. Medizinische Vorrichtung nach Anspruch 5 oder 15, wobei das distale Ende des proximalen Abschnitts und das proximale Ende des distalen Abschnitts so zusammengefügt sind, daß sie einen Stumpfstoß im Verbinderteil bilden.

22. Medizinische Vorrichtung nach Anspruch 5 oder 15 bis 20, wobei das distale Ende des proximalen Abschnitts ein zulaufendes Teil bildet und das proximale Ende des distalen Abschnitts ein zulaufendes Teil bildet und sich die zulaufenden Teile mindestens teilweise überlappen.

23. Medizinische Vorrichtung nach einem der Ansprüche 1 bis 22, wobei der längliche Abschnitt ein Metall oder eine Metallegierung aufweist und optional das Metall oder die Metallegierung Edelstahl, Nickel-Titan-Legierung, Nickel-Chrom-Legierung, Nickel-Chrom-Eisen-Legierung, Kobaltlegierung oder deren Kombinationen aufweist.

24. Medizinische Vorrichtung nach einem der Ansprüche 5 oder 15 bis 22, wobei die medizinische Vorrichtung ferner eine Außenstruktür aufweist, die um mindestens einen Teil des distalen Abschnitts angeordnet ist.

25. Medizinische Vorrichtung nach einem der Ansprüche 1 bis 24, wobei das Strukturteil ein Metall oder eine Metalllegierung aufweist und optional das Metall oder die Metallegierung Edelstahl, Nickel-Titan-Legierung, Nickel-Chrom-Legierung, Nickel-Chrom-Eisen-Legierung, Kobaltlegierung, Nickel oder deren Kombinationen aufweist.

26. Medizinische Vorrichtung nach einem der Ansprüche 1 bis 24, wobei das Strukturteil ein Polymer oder einen Metall-Polymer-Verbundstoff aufweist.

27. Medizinische Vorrichtung nach einem der Ansprüche 5 oder 15 bis 22, wobei die Verbinderstruktur ein Röhrenteil aufweist, das um das distale Ende des proximalen Abschnitts und das proximale Ende des distalen Abschnitts angeordnet ist.

28. Medizinische Vorrichtung nach einem der Ansprüche 3 bis 5 oder 15 bis 22, wobei der zweite längliche Abschnitt ein Metall oder eine Metallegierung aufweist und optional das Metall oder die Metallegierung Edelstahl, Nickel-Titan-Legierung, Nickel-Chrom-Legierung, Nickel-Chrom-Eisen-Legierung, Kobaltlegierung oder deren Kombinationen aufweist.

29. Medizinische Vorrichtung nach einem der Ansprüche 1 bis 28, wobei die medizinische Vorrichtung einen Führungsdraht aufweist.

30. Führungsdraht mit:
einem proximalen Abschnitt mit einem distalen Ende;
einem distalen Abschnitt mit einem proximalen Ende; und
einer Verbinderstruktur, die benachbart zum distalen Ende des proximalen Abschnitts und zum proximalen Ende des distalen Abschnitts angeordnet ist; und
einer Einrichtung zum Ausüben einer Druckkraft auf die Verbinderstruktur und die Enden des proximalen und distalen Abschnitts, um für eine mechanische Verriegelung zwischen der Verbinderstruktur und dem proximalen und distalen Abschnitts zu sorgen, wobei die Einrichtung ein Verbindermaterial aus Wismutlegierung aufweist.

31. Verfahren zur Herstellung der medizinischen Vorrichtung nach einem der Ansprüche 1 bis 29, wobei das Verfahren die folgenden Schritte aufweist:
Bereitstellen des länglichen Abschnitts mit dem Ende;
Bereitstellen des Strukturteils, das die Öffnung darin bildet;
Anordnen des Endes des länglichen Abschnitts in der Öffnung;
Anordnen des Verbindermaterials aus Wismutlegierung in der Öffnung; und
Erstarren- und Ausdehnenlassen des Verbindermaterials aus Wismutlegierung, um eine Druckkraft in der Öffnung im Strukturteil auszuüben.

32. Verfahren zur Herstellung der medizinischen Vorrichtung nach einem der Ansprüche 3 bis 5, 15 bis 24 oder 27 bis 28, wobei das Verfahren die folgenden Schritte aufweist:
Bereitstellen des ersten länglichen Abschnitts mit dem Ende;
Bereitstellen des zweiten länglichen Abschnitts mit dem Ende;
Bereitstellen des Strukturteils, das die erste Öffnung und zweite Öffnung darin bildet;
Anordnen des Endes des ersten länglichen Abschnitts in der ersten Öffnung;
Anordnen des Endes des zweiten länglichen Abschnitts in der zweiten Öffnung;
Anordnen des Verbindermaterials aus Wismutlegierung in der ersten Öffnung und der zweiten Öffnung; und
Erstarren- und Ausdehnenlassen des Verbindermaterials aus Wismutlegierung, um eine Druckkraft in der ersten
Öffnung und der zweiten Öffnung im Strukturteil auszuüben.

33. Verfahren nach Anspruch 32, wobei die erste Öffnung und die zweite Öffnung Öffnungen an den entgegengesetzten Enden eines gemeinsamen Lumens sind, das im Strukturteil gebildet ist.

34. Verfahren nach Anspruch 32 oder 33, wobei die medizinische Vorrichtung einen Führungsdraht aufweist.

## Revendications

1. Dispositif médical comprenant :
une section allongée possédant une extrémité ;
un membre de structure définissant une ouverture en son sein, l'extrémité de la section allongée s'étendant dans l'ouverture ; et
une matière de raccord constituée d'un alliage à base de bismuth, disposée au sein de l'ouverture, la matière de raccord étant configurée pour se dilater à l'état solidifié afin d'exercer une force de compression au sein de l'ouverture dans le membre de structure.

2. Dispositif médical selon la revendication 1, dans lequel la matière de raccord constituée d'un alliage à base de bismuth s'est solidifiée et s'est dilatée pour exercer une force de compression sur le membre de structure et sur l'extrémité de la section allongée pour obtenir un verrouillage réciproque mécanique entre le membre de structure et la section allongée.

3. Dispositif médical selon la revendication 1, dans lequel le membre de structure définit une deuxième ouverture en son sein, le dispositif médical englobant en outre une deuxième section allongée possédant une extrémité, l'extrémité de la deuxième section allongée s'étendant dans la deuxième ouverture ; et
dans lequel une matière de raccord constituée d'un alliage à base de bismuth est disposée au sein de la deuxième ouverture, la matière de raccord étant configurée pour se dilater à l'état solidifié afin d'exercer une force de compression au sein du membre de structure.

4. Dispositif médical selon la revendication 3, dans lequel la première ouverture et la deuxième ouverture sont des ouvertures situées aux extrémités opposées d'une lumière commune définie dans le membre de structure.

5. Dispositif médical selon revendication 4, dans lequel : la première section allongée comprend une section proximale possédant une extrémité distale ; la deuxième section allongée comprend une section distale possédant une extrémité proximale ; et le membre de structure comprend un membre faisant office de raccord définissant la lumière possédant la première ouverture et la deuxième ouverture, l'extrémité distale de la section proximale s'étendant dans la lumière à travers la première ouverture et l'extrémité proximale de la section distale s'étendant dans la lumière à travers la deuxième ouverture ; et la matière de raccord constituée d'un alliage à base de bismuth est disposée au sein de la lumière, la matière de raccord étant configurée pour se dilater à l'état solidifié afin d'exercer une force de compression au sein du membre faisant office de raccord.

6. Dispositif médical selon l'une quelconque des revendications 1 à 5, dans lequel la matière de raccord constituée d'un alliage à base de bismuth possède un point de fusion eutectique dans la plage de 200 °C ou moins, ou bien, en variante, la matière de raccord constituée d'un alliage à base de bismuth possède un point de fusion eutectique dans la plage de 150 °C ou moins.

7. Dispositif médical selon l'une quelconque des revendications 1 à 6, dans lequel la matière de raccord constituée d'un alliage à base de bismuth comprend du bismuth dans la plage de 4 à 80 % en poids.

8. Dispositif médical selon l'une quelconque des revendications 1 à 7, dans lequel la matière de raccord constituée d'un alliage à base de bismuth comprend du bismuth et un élément d'alliage supplémentaire choisi parmi le groupe comprenant de l'étain, de l'indium, ou encore leurs mélanges.

9. Dispositif médical selon l'une quelconque des revendications 1 à 8, dans lequel la matière de raccord constituée d'un alliage à base de bismuth comprend du bismuth dans la plage de 35 à 45 % en poids et de l'étain dans la plage de 55 à 65 % en poids, ou bien, en variante, la matière de raccord constituée d'un alliage à base de bismuth comprend du bismuth dans la plage de 53 à 63 % en poids et de l'étain dans la plage de 37 à 47 % en poids.

10. Dispositif médical selon l'une quelconque des revendications 1 à 9, dans lequel la matière de raccord constituée d'un alliage à base de bismuth comprend un alliage eutectique de bismuth-étain.

11. Dispositif médical selon l'une quelconque des revendications 1 à 8, dans lequel la matière de raccord constituée d'un alliage à base de bismuth comprend du bismuth dans la plage de 2 à 10 % en poids et de l'indium dans la plage de 90 à 98 % en poids, ou bien, en variante, la matière de raccord constituée d'un alliage à base de bismuth comprend du bismuth dans la plage de 62 à 72 % en poids et de l'indium dans la plage de 28 à 38 % en poids, ou bien, en variante, la matière de raccord constituée d'un alliage à base de bismuth comprend du bismuth dans la plage de 29 à 39 % en poids et de l'indium dans la plage de 61 à 71% en poids.

12. Dispositif médical selon l'une quelconque des revendications 1 à 8 ou 11, dans lequel la matière de raccord constituée d'un alliage à base de bismuth comprend un alliage eutectique de bismuth-indium.

13. Dispositif médical selon l'une quelconque des revendications 1 à 8, dans lequel la matière de raccord constituée d'un alliage à base de bismuth comprend du bismuth dans la plage de 53 à 63 % en poids, de l'indium dans la plage de 20 à 30 % en poids, et de l'étain dans la plage de 12 à 22 % en poids.

14. Dispositif médical selon une quelconque des revendications 1 à 8 ou 13, dans lequel la matière de raccord constituée d'un alliage à base de bismuth comprend un alliage eutectique de bismuth-indium-étain.

15. Dispositif médical selon la revendication 5, dans lequel la matière de raccord constituée d'un alliage à base de bismuth s'est solidifiée et s'est dilatée pour exercer une force de compression sur le membre faisant office de raccord et sur l'extrémité distale de la section proximale et sur l'extrémité proximale de la section distale pour obtenir un verrouillage réciproque mécanique entre le membre faisant office de raccord et les extrémités des sections allongées.

16. Dispositif médical selon la revendication 5 ou 15, dans lequel la section proximale possède une première flexibilité et la section distale possède une deuxième flexibilité, et dans lequel l'extrémité distale de la section proximale et l'extrémité proximale de la section distale se chevauchent pour définir une zone dans laquelle la première flexibilité se confond avec la deuxième flexibilité.

17. Dispositif médical selon la revendication 5, 15 ou 16, dans lequel l'extrémité distale de la section proximale possède une dimension réduite et l'extrémité proximale de la section distale possède une dimension réduite.

18. Dispositif médical selon la revendication 17, dans lequel les portions de dimension réduite possèdent un profil uniforme.

19. Dispositif médical selon la revendication 17, dans lequel les portions de dimension réduite possèdent une conicité.

20. Dispositif médical selon la revendication 17, dans lequel les portions de dimension réduite possèdent une configuration permettant un verrouillage réciproque.

21. Dispositif médical selon la revendication 5 ou 15, dans lequel l'extrémité distale de la section proximale et l'extrémité proximale de la section distale sont jointes pour définir un joint d'about au sein du membre faisant office de raccord.

22. Dispositif médical selon l'une quelconque des revendications 5 ou 15-20, dans lequel l'extrémité distale de la section proximale définit une portion de forme tronconique et l'extrémité proximale de la section distale définit une portion de forme tronconique, les portions de forme tronconique se chevauchant l'une l'autre au moins en partie.

23. Dispositif médical selon l'une quelconque des revendications 1-22, dans lequel la section allongée comprend un métal ou un alliage métallique et, le cas échéant, le métal ou l'alliage métallique comprend de l'acier inoxydable, un alliage de nickel-titane, un alliage de nickel-chrome, un alliage de nickel-chrome-fer, un alliage à base de cobalt, ou encore leurs combinaisons.

24. Dispositif médical selon l'une quelconque des revendications 5 ou 15-22, dans lequel le dispositif médical englobe en outre une structure externe disposée autour d'au moins une portion de la section distale.

25. Dispositif médical selon l'une quelconque des revendications 1-24, dans lequel le membre de structure comprend un métal ou un alliage métallique et, le cas échéant, le métal ou l'alliage métallique comprend de l'acier inoxydable, un alliage de nickel-titane, un alliage de nickel-chrome, un alliage de nickel-chrome-fer, un alliage à base de cobalt, du nickel, ou encore leurs combinaisons.

26. Dispositif médical selon l'une quelconque des revendications 1-24, dans lequel le membre de structure comprend un polymère ou un composite métal-polymère.

27. Dispositif médical selon l'une quelconque des revendications 5 ou 15-22, dans lequel la structure faisant office de raccord comprend un membre tubulaire disposé autour de l'extrémité distale de la section proximale et autour de l'extrémité proximale de la section distale.

28. Dispositif médical selon l'une quelconque des revendications 3 à 5 ou 15-22, dans lequel la deuxième section allongée comprend un métal ou un alliage métallique et, le cas échéant, le métal ou l'alliage métallique comprend de l'acier inoxydable, un alliage de nickel-titane, un alliage de nickel-chrome, un alliage de nickel-chrome-fer, un alliage à base de cobalt, ou encore leurs combinaisons.

29. Dispositif médical selon l'une quelconque des revendications 1-28, dans lequel le dispositif médical comprend un fil guide.

30. Fil guide comprenant :
une section proximale possédant une extrémité distale ;
une section distale possédant une extrémité proximale ; et
une structure faisant office de raccord disposée en position adjacente à l'extrémité distale de la section proximale et à l'extrémité proximale de la section distale ; et
un moyen pour exercer une force de compression sur la structure faisant office de raccord et sur les extrémités des sections proximale et distale afin d'obtenir un verrouillage réciproque mécanique entre la structure faisant office de raccord et les sections proximale et distale, le moyen englobant une matière de raccord constituée d'un alliage à base de bismuth.

31. Procédé de fabrication du dispositif médical selon l'une quelconque des revendications 1-29, le procédé comprenant le fait de :
procurer la section allongée possédant l'extrémité ;
procurer le membre de structure définissant l'ouverture en son sein ;
disposer l'extrémité de la section allongée dans l'ouverture ;
disposer la matière de raccord constituée d'un alliage à base de bismuth au sein de l'ouverture ; et
laisser la matière de raccord constituée d'un alliage à base de bismuth se solidifier et se dilater pour exercer une force de compression au sein de l'ouverture dans le membre de structure.

32. Procédé de fabrication du dispositif médical selon l'une quelconque des revendications 3-5, 15-24 ou 27-28, le procédé comprenant le fait de :
procurer la première section allongée possédant l'extrémité :
procurer la deuxième section allongée possédant l'extrémité ;
procurer le membre de structure définissant la première ouverture et la deuxième ouverture en son sein;
disposer l'extrémité de la première section allongée dans la première ouverture ;
disposer l'extrémité de la deuxième section allongée dans la deuxième ouverture ;
disposer la matière de raccord constituée d'un alliage à base de bismuth au sein de la première ouverture et au sein de la deuxième ouverture ; et
laisser la matière de raccord constituée d'un alliage à base de bismuth se solidifier et se dilater pour exercer une force de compression au sein de la première ouverture et au sein de la deuxième ouverture dans le membre de structure.

33. Procédé selon la revendication 32, dans lequel la première ouverture et la deuxième ouverture sont des ouvertures situées aux extrémités opposées d'une lumière commune définie dans le membre de structure.

34. Procédé selon la revendication 32 ou 33, dans lequel le dispositif médical comprend un fil guide.
